# EUROPEAN PATENT SPECIFICATION

(11) **EP 1 842 546 B1**
(45) Date of publication and mention of the grant of the patent: **11.03.2009**
(21) Application number: 06450052.3
(22) Date of filing: 04.04.2006
(51) Int. Cl.: A61K 31/737, A61P 31/16

(54) **Cellulose sulfate for the treatment of rhinovirus infection**
Zellulosesulfat zur Behandlung von Rhinovirus-Infektionen
Sulfate de la cellulose pour le traitment des infections par rhinovirus

(43) Date of publication of application: 10.10.2007
(73) Proprietor: Marinomed Biotechnologie GmbH, 1210 Vienna (AT)
(72) Inventor: Grassauer, Andreas, Dr., 1220 Vienna (AT)
(74) Representative: Sonn & Partner Patentanwälte

(56) References cited:
- WO-A-97/12621
- WO-A-20/05004882
- US-A1- 2003 181 415
- US-A1- 2005 232 895
- GIROUD J P ET AL: "RELATIONS ENTRE ACTIVITE ANTI-INFLAMMATOIRE ET ANTICOMPLEMENTAIRE DE QUELQUES POLYSACCHARIDES SULFATES DOUES DE PROPRIETES ANTICOAGULANTES" THERAPIE, DOIN, PARIS, FR, vol. 28, no. 5, 1973, pages 889-905, XP009069780 ISSN: 0040-5957
- SILVA M R E ET AL: "ANTI-INFLAMMATORY ACTION OF SULFATED POLUSACCHARIDES" BIOCHEMICAL PHARMACOLOGY, PERGAMON, OXFORD, GB, vol. 18, no. 6, 1969, pages 1285-1295, XP000952171 ISSN: 0006-2952
- CHARLES C H ET AL: "RECENT ADVANCES IN RHINOVIRUS THERAPEUTICS" CURRENT DRUG TARGETS. INFECTIOUS DISORDERS, BENTHAM SCIENCE PUBLISHERS, HILVERSUM, NL, vol. 4, no. 4, 2004, pages 331-337, XP009070283 ISSN: 1568-0053
- KIM MI-SUN ET AL: "Inhibitory effect of water-soluble chitosan on TNF-alpha and IL-8 secretion from HMC-1 cells" IMMUNOPHARMACOLOGY AND IMMUNOTOXICOLOGY, vol. 26, no. 3, 2004, pages 401-409, XP009071536 ISSN: 0892-3973

## Description

The present invention relates to the field of immunology and antiviral agents.

Picornaviruses represent a very large virus family of small ribonucleic acid-containing viruses responsible for many serious human and animal diseases. Picornaviruses include four major groups: enteroviruses, rhinoviruses, cardioviruses and apthoviruses.

The human rhinoviruses consist of at least 100 serotypes and are the primary causative agents of the common cold. Because of the large number of serotypes, development of a vaccine is problematic; antiviral agents may therefore be the best approach to treatment. Rhinoviruses are composed of a surrounding capsid, which contains four viral proteins VP1, VP2, VP3 and VP4. Proteins VP1, VP2 and VP3 are organised into 60 repeating protameric icosahedral units. These are thought to be the cause of antigen diversity associated with these viruses.

Present treatment approaches include the application of rhinovirus specific RNA, as disclosed in the DE 19825395, which binds to the canyon region of the capsid, which is necessary to host receptor (e.g. ICAM-1, intercelluar adhesion molecule-1) binding and cell infection.

Another method comprises the administration of soluble ICAM-1 proteins or derivatives of ICAM-1 as disclosed in the US 6,326,004 and the US 6,096,862 (tICAM-1, truncated ICAM-1) to neutralize viral particles (virions).

Chemical compounds with antiviral activity against rhinoviruses are disclosed in the EP 0523803.

The WO 97/12621 A discloses the use of cellulose sulfate against HIV, HSV, and diverse bacteria strains. Therein it is comtemplated to use cellulose sulfate for vaginal administration to prevent infections.

Giroud et al., Thérapie 28(5) (1973), 889-905, discloses apparent anti-inflammatory and anti-complementary effects of five sulfated polysaccharids. Cellulose sulfate is disclosed to be effective against carragenine induced oedema.

It is a goal of the present invention to provide a pharmaceutical composition for effective treatment of rhinitis.

The present invention provides the use of a polymer for the manufacture of an antiviral pharmaceutical composition for the treatment of rhinovirus infections (rhinitis), wherein the polymer is cellulose sulfate. Cellulose sulfate as such is known in the state of the art. The manufacture of cellulose sulfate is e.g. disclosed in the DD 299313.

As disclosed in US 2003/181415 A sulfated polysaccharides, including cellulose sulfate, are known to be effective against various enveloped viruses and in particular herpes simplex virus (HSV), Papilloma viruses and HIV. Cellulose sulfate binds to the lipid membrane of encapsulated viruses and prevents fusion to and infection of host cells. The preparation disclosed therein is used to prevent sexually transmitted infections. Contrary to the US 2003/181415 A it was now surprisingly found that cellulose sulfate is not only effective against encapsulated viruses but also against picornaviruses, including rhinoviruses. Prior to the present invention it was believed that cellulose sulfate only binds to the lipid membrane of encapsulated viruses and prevents fusion to and infection of host cells. The viral particles of the family of picornaviruses, of which rhinovirus is a member, are not enveloped and are icosahedral in structure, composed of encapsulating capsid proteins.

Rhinovirus infections lead to the common cold with symptoms such as fever, cough, and nasal congestion. These symptoms are caused by an over or unspecific reaction of the immune system. Therefore common treatment forms of a rhinovirus infection include administration of analgesics such as aspirin or acetaminophen / paracetamol, as well as localised versions targeting the throat (often delivered in lozenge form), nasal decongestants which reduce the inflammation in the nasal passages by constricting local blood vessels, cough suppressants (which work to suppress the cough reflex of the brain or by diluting the mucus in the lungs), and first-generation anti-histamines such as brompheniramine, chlorpheniramine, and clemastine (which reduce mucus gland secretion and thus combat blocked/runny noses but also may make the user drowsy).

It has now been surprisingly found that the polymer of the present invention, cellulose sulfate, has anti-inflammatory activity as well and is thus capable to act against typical rhinovirus infection symptoms. Therefore the use of a polymer for the manufacture of a pharmaceutical composition for the treatment of the symptoms of a rhinovirus infection, wherein the polymer is cellulose sulfate is also contemplated. It has been demonstrated herein that cellulose sulfate shows significant anti-inflammatory action over cellulose, which has no immunomodulating effects. It was completely surprising that such an effect could be achieved through sulfatation of cellulose. E.g. in the US 2006/0040896 A a heparin (a sulfated carbohydrate polymer of the glycosaminoglycan family, obtainable from liver) derivative is disclosed, wherein the anti-inflammatory efficacy was increased by desulfation.

Without being limited to a specific theory it is assumed that the anti-inflammatory polymer of the present invention, e.g. cellulose sulfate binds to (and antagonizes) specific receptors of immune cells and prevents TNF-alpha production or release. TNF-alpha acts as activator for T-cells which in turn would produce IFN-gamma.

Also disclosed herein is the use of chitosan as antiviral and anti-inflammatory polymer. Chitosan is a linear polysaccharide composed of randomly distributed β-(1-4)-linked D-glucosamine (deacetylated unit) and N-acetyl-D-glucosamine (acetylated unit). Chitosan is produced commercially by deacetylation of chitin, which is the structural element in the exoskeleton of crustaceans (crabs, shrimp, etc.). There is a great diversity among chitosan products depending on the degree of deacetylation and origin of chitin. Most chitosan preparations are hardly soluble in neutral pH conditions. However, especially preferred is the use of water soluble chitosan. The production of water soluble chitosan is not trivial, but has already been demonstrated by Jakwang Co Ltd (KR 2001/027073, KR 2003/079444, US 2002/155175), which is a commercial supplier for water soluble chitosan. Water soluble chitosan is preferably completely soluble without the formation of colloids or gelatinous phases. Preferably the polymer is >90%, in particular preferred > 99% soluble (of its initial weight). Specifically preferred chitosan preparations, but also the general polymer preparations, are purified or purifyable by passage through a 0.22 µm pore sterilisation filter.

Therefore in a preferred embodiment of the present invention the polymer is purified, in particular sterilely purified, e.g. through filtration through a sterilisation filter in soluble form.

Preferably the pharmaceutical preparation is in form of a preparation for topical or mucosal use, preferably skin lotions, cremes, sprays or gargle solutions. Preparations for mucosal application include nose sprays or drops, e.g. in form of a fluid solution, or other nasal drug delivery systems known in the state of the art, e.g. from the US 6,391,452. The polymer is especially suitable for topical application to treat skin or mucosal inflammation. But also systemic, e.g. parenteral or oral (also for specific mucosal treatment), is possible, especially for low molecular weight polymers. The present invention also provides the use of the pharmaceutical preparations.

Preferably, the polymer has a molecular weight ranging from about 15000 to 3,000,000 Da. Preferably, the molecular weight is greater than about 500,000, or for systemic administration lower than 500,000 Da.

Also preferred is that the preparation comprises pharmaceutical carriers or additives. The term "carrier" refers to a diluent, e.g. water, saline, excipient, or vehicle with which the composition can be administered. For a solid or fluid composition the carriers or additives in the pharmaceutical composition may comprise SiO₂, TiO₂, a binder, such as microcrystalline cellulose, polyvinylpyrrolidone (polyvidone or povidone), gum tragacanth, gelatine, starch, lactose or lactose monohydrate, alginic acid, maize starch and the like; a lubricant or surfactant, such as magnesium stearate, or sodium lauryl sulphate; a glidant, such as colloidal silicon dioxide; a sweetening agent, such as sucrose or saccharin. Preferably the preparation comprises buffers or pH adjusting agents, e.g. selected from citric acid, acetic acid, fumaric acid, hydrochloric acid, malic acid, nitric acid, phosphoric acid, propionic acid, sulfuric acid, tartaric acid, or combinations thereof. Cellulose sulfate in the form of a pharmaceutically acceptable salt, for example sodium cellulose sulfate may also be used. Other pharmaceutically acceptable salts include, among others, potassium, lithium and ammonium cellulose sulfate.

The degree of sulfation of cellulose sulfate is preferably above 12% and most preferably about 17-18%, or maximal sulfation.

The polymer is preferably a homopolymer, e.g. pure cellulosesulfate, or alternatively a derivative heteropolymer.

Furthermore, preferably the polymer is in amounts between 0.01% and 20%, preferably between 0.1% and 10%, most preferred between 0.5% and 5% of the preparation (%-values are given in w/w-%). In the pharmaceutical preparation also other anti-viral polymers than cellulose sulfate can be present, most preferably water soluble chitosan. For these polymers the same preferred concentration ranges apply.

The administration of the preparation is not limited to administrations at the same time of a disease but can also be used before or after an infection, e.g. for prophylactic treatment, i.e. a treatment before an expected infection to reduce the force of the disease.

In the preparation only one anti-viral agent, e.g. cellulose sulfate, may be present. Nevertheless, in a more preferred embodiment the pharmaceutical preparation comprises at least two different anti-viral components, preferably one component is chitosan. Chitosan, especially water soluble chitosan (Jakwang), is an exceptional anti-viral polymer (alone or in combination), which can be used in a preparation of the present invention.

The present invention is further illustrated by the following figures and examples.

### Figures:

Fig. 1: Dose-activity curve of cellulose sulfate. In a standardized testing procedure cellulose sulfate was active against human Rhinovirus at an effective concentration 50 (EC50) of 17.5µg/ml. The Y-Axis shows the optical density as determined in a neutral red test. The X-axis shows the polymer concentration in µg/ml.
Fig. 2: LPS induced TNF-alpha production is inhibited by Cellulose sulphate and water soluble Chitosan but not other biopolymers.
   The y-axis shows the amount of TNF-alpha in pg/ml that is produced after 18hours of incubation with LPS and the corresponding biopolymer. The black bars correspond to a test substance concentration of 330µg/ml. The numbers at the x-axis indicate the corresponding Biopolymers whereas 1 is Carboxymethylcellulose T70 (Niklacell, Mare GmbH, Austria), 2 is water soluble Chitosan (Jakwang, Korea), 3 Carboxymethylchitosan, 4 is Carrageenan (Carbamer), 5 is Carboxymethylcellulose T35 (Niklacell, Mare GmbH, Austria), 6 is kappa Carrageenan (Sigma), 7 is lambda Carrageenan (Sigma), 8 is cellulose sulfate (Acros), 9 uninduced not treated blood, 10 LPS induced blood and 11 is the Dexamethasone (Sigma) reference control 100ng/ml.

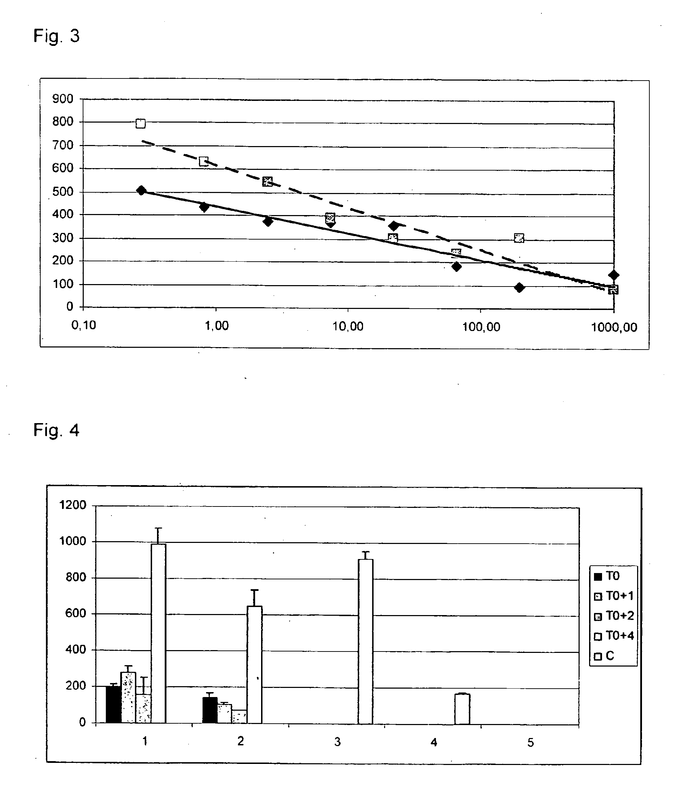

Fig. 3: The inhibition of TNF-alpha production by Cellulose sulphate and Chitosan is dose dependent
   The y-axis shows the amount of TNF-alpha in pg/ml produced after 18hours of incubation with LPS and the corresponding biopolymer. The X-axis shows the test substance concentration in µg/ml at a logarithmic scale. Cellulose sulphate data is shown in diamonds and the corresponding fit as black line. Chitosan is shown as grey squares and the fit as dashed line.
Fig. 4: Cellulose Sulphate and Chitosan are active by administration hours after stimulation
   The y-axis shows the amount of TNF-alpha in pg/ml produced after 18hours of incubation with LPS and the corresponding biopolymer at different time points. The black bars indicate the addition of the test substance at the same time with LPS. For grey bar data the test substance was added 1h after the stimulus, dashed bars 2 hours after stimulus and white bars 4 hours after stimulus. Lane number 1 on the x-axis corresponds to Cellulose sulphate and number 2 to chitosan both at a concentration of 20µg/ml. The wave shaped bars in lanes 3, 4 and 5 indicate the assay controls which are indicated as with LPS stimulus without substance, LPS + Dexamethasone reference substance and unstimulated blood respectively.

### Examples:

### Example 1: Activity of cellulose sulfate against human rhinovirus

The following test essentially was described by Sidwell and Huffman (1) as modified and described by Barnard et al., (2). The test was performed at the University of Utah in the laboratory of Dr. Robert Sidwell.

The following cells and media were used: HeLa-Ohio 1 cells (human cervical carcinoma), obtained from Dr. Fred Hayden (University of Virginia). Growth medium for cells were Dulbecco's modified Eagle medium (DMEM) with high glucose (GIBCO, Grand Island, NY) with 10% fetal bovine serum (FBS) obtained from HyClone Laboratories (Logan, UT) and 0.1% NaHCO3. Growth medium not supplemented with antibiotics. Test medium for RV-2 were the same as for the cells, but with 10 mM MgCl2 also included in the medium. Rhinovirus type 2 (RV-2), strain HGP was used. Preparation of Compounds for Testing:

Compounds were weighed and added in a sufficient amount to the test medium (lacking serum) to obtain 2000 µg/ml as the highest concentration. Three or seven 0.5 log10 dilutions from the highest concentration used were made to obtain four or eight concentrations of each compound for testing.

### Antiviral Testing Procedure

Cells were seeded into 96-well flat-bottomed tissue culture plates (Corning Glass Works, Corning, NY), 0.2 ml/well, at the proper cell concentration, and incubated overnight at 37°C in order to establish a cell monolayer. When the monolayer was established, the growth medium was decanted and the various dilutions (either four or eight) of test compound were added to each well (three wells/dilution, 0.1 ml/well). Compound diluent medium was added to cell and virus control wells (0.1 ml/well). Virus, diluted in test medium, was added to compound test wells (3 wells/dilution of compound) and to six virus control wells at 0.1 ml/well. Virus was added approximately 5 min after compound. Test medium without virus was added to all toxicity control wells (two wells/dilution of each test compound) and to cell six control wells at 0.1 ml/well. The plates were then incubated at 33°C in a humidified incubator with 5% CO2, 95% air atmosphere until virus control wells have adequate cytopathic effect (CPE) readings. This was achieved after 96hr exposure of cells to virus. Cells were then examined microscopically for CPE. The 50% effective concentration (EC50) and 50% cytotoxicconcentration (IC50) were calculated by regression analysis of the virus CPE data and the cytotoxicity data, respectively. A known active substance was run in the same manner as above for each batch of compounds tested. For RV-2, pirodavir (ethyl 4-[2-[1-(6-methyl-3-pyridazinyl)-4-piperidinyl]ethoxy]benzoate) was used.

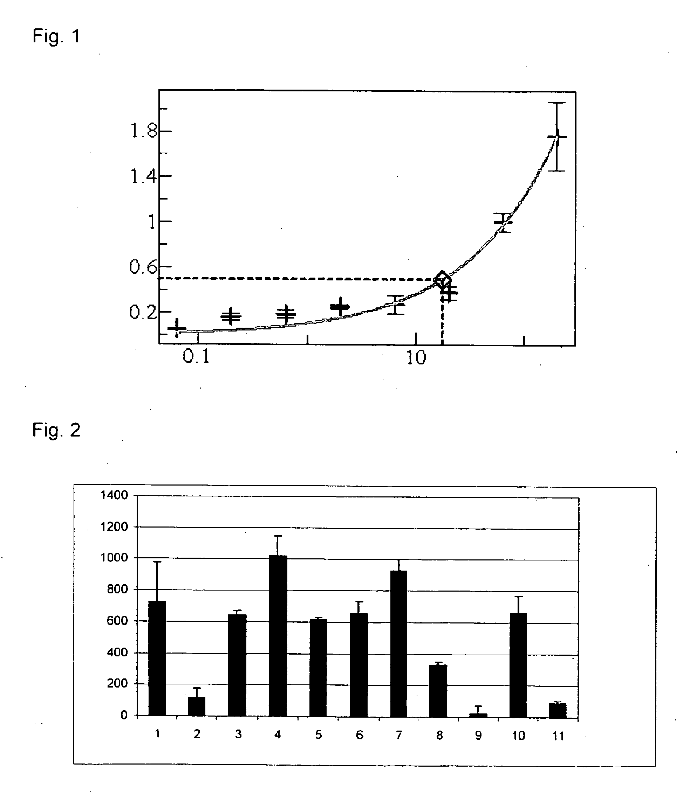

After the plates were read visually for virus cytopathology and compound cytotoxicity, the cells were stained with neutral red (fig. 1) to verify the visual determinations. 0.1 ml of sterile neutral red (0.034% in physiological saline solution) were added to each well, the plates were wrapped in aluminum foil to minimize exposure to light, and then placed in an incubator at 37°C for two hours. All medium was then removed and the cells gently washed 2x (0.2 ml/well for each wash) with phosphate buffered saline. The plates were inverted and allowed to drain on a paper towel. Neutral red taken up into cells was extracted from the cells by adding 0.2 ml of an equal volume mixture of absolute ethanol and Sörensen citrate buffer, pH 4, to each well and placing the plates in the dark at room temperature for 30 minutes. The contents of each was mixed gently immediately to prior obtaining the O.D. values of each well by reading the plates at 540 nm with a Model EL309 microplate reader (Bio-Tek Instruments, Inc., Winooski, VT). The EC50 and CC50 were calculated by regression analysis.

### Example 2: Anti-inflammatory effect of cellulose sulfate

The inducing or inhibitory effects of cellulose sulfate and other biopolymers were investigated using primary blood cells in combination with cytokine ELISAs such as TNF-alpha. The cells were either left non-stimulated or they were induced with 200 ng/ml LPS (Lipopolysaccharide). The cells were stimulated for 18h and TNF-alpha secretion was measured by using a commercial TNF-alpha ELISA (R&D systems). The results are given in figs. 2-4.

As it can be seen in Figure 2 Chitosan (2) and cellulose sulphate (9) have a TNF-alpha inhibition activity. Other polymers do not have a significant inhibiting activity.

As shown in Figure 3 the TNF-alpha inhibiting effect of Cellulose sulphate and Chitosan is dose dependent and the two polymers inhibit at comparable concentrations.

As shown in Figure 4 Cellulose Sulphate and Chitosan are active up to 2 hours after stimulation. 2 hours after stimulation both polymers were as active as when added at the same time with LPS. When the polymers are added 4 hours after the stimulus the inhibitory effect is dramatically reduced.

### Example 3: Activity of cellulose sulphate and other biopolymers on immune cells

Cellulose sulfate was investigated concerning its potential activating or inhibiting function on primary human blood cells. Several experiments that measure the induction of cytokines were performed that suggest that cellulose sulfate on its own is neither immunostimulatory on blood cells nor on isolated lymphocytes. In contrast, cellulose sulfate markedly reduced the production of the pro-inflammatory cytokine TNF-alpha in LPS stimulated primary blood cells. Therefore, it is concluded that cellulose sulfate acts as a novel anti-inflammatory substance.

In an analogous experiment the immuno-stimulatory capacity of cellulose sulfate was determined. Cells were either left non-stimulated, activated with PMA + ionomycin or LPS or treated with 2 different batches of cellulose sulfate. Both batches did not lead to any induction of TNF-alpha release. In a similar setting also the cytokines IL-1 beta, IL-6, and IL-12 p40 were tested with identical results. Primary lymphocytes were tested in a similar setting for the capacity of cellulose sulfate to induce the release of IL-2 or IFN-gamma. Again no cytokine stimulation was detected.

Taken together the data indicate that cellulose sulfate does not provoke a cytokine release of primary blood cells or primary lymphocytes in-vitro, but exhibits a remarkable inhibitory effect on LPS stimulated primary blood cells.

### References

1. Sidwell, R.W. and J.H. Huffman. 1971. Use of disposable micro tissue culture plates for antiviral and interferon induction studies. Appl. Microbiol. 22:797-801.
2. Barnard, D. L., Hubbard, V. D., Smee, D. F., Sidwell, R. W., Watson, K. G. W., Tucker, S. P. T., Reece, P. A. R. 2004. In Vitro Activity of Expanded-Spectrum Pyridazinyl Oxime Ethers Related to Pirodavir: Novel Capsid-Binding Inhibitors with Potent Antipicornavirus Activity. Antimicrob. Agents Chemother. 48:1766-1772.

## Claims

1. Use of a polymer for the manufacture of an antiviral pharmaceutical composition for the treatment of a rhinovirus infection, wherein the polymer is cellulose sulfate.

2. Use according to claim 1, **characterized in that** the preparation is in form of a preparation for topical or mucosal use, preferably nose sprays, drops or gargle solutions.

3. Use according to claim 1 or 2, **characterized in that** the preparation comprises pharmaceutical carriers or additives.

4. Use according to any one of claims 1 to 3, **characterized in that** the polymer is in amounts between 0,01% and 20%, preferably between 0,1% and 10%, most preferred between 0,5% and 5% of the preparation.

5. Use according to any one of claims 1 to 4, **characterized in that** the preparation comprises at least two different antiviral components, preferably one component is chitosan.

6. Use according to any one of claims 1 to 5, **characterized in that** the preparation is sterile.

## Patentansprüche

1. Verwendung eines Polymers bei der Herstellung einer antiviralen pharmazeutischen Zusammensetzung für die Behandlung einer Rhinovirus-Infektion, wobei das Polymer Cellulose-Sulfat ist.

2. Verwendung nach Anspruch 1, **dadurch gekennzeichnet, dass** die Präparation in Form einer Präparation zur topischen und mukosalen Verwendung, vorzugsweise als Nasenspray, Tropfen oder Gurgellösung, ist.

3. Verwendung nach Anspruch 1 oder 2, **dadurch gekennzeichnet, dass** die Präparation pharmazeutische Träger oder Zusatzstoffe enthält.

4. Verwendung nach einem der Ansprüche 1 bis 3, **dadurch gekennzeichnet, dass** das Polymer in Mengen zwischen 0,01 % und 20%, vorzugsweise 0,1 % und 10 %, am meisten bevorzugt zwischen 0,5 % und 5 % der Präparation vorliegt.

5. Verwendung nach einem der Ansprüche 1 bis 4, **dadurch gekennzeichnet, dass** die Präparation zumindest zwei unterschiedliche antivirale Komponenten umfasst, wobei eine Komponente vorzugsweise Chitosan ist.

6. Verwendung nach einem der Ansprüche 1 bis 5, **dadurch gekennzeichnet, dass** die Präparation steril ist.

## Revendications

1. Utilisation d'un polymère pour fabriquer une composition pharmaceutique antivirale pour le traitement d'une infection par un rhinovirus, dans laquelle le polymère est le sulfate de cellulose.

2. Utilisation selon la revendication 1, **caractérisée en ce que** la préparation se présente sous la forme d'une préparation pour utilisation par voie topique ou muqueuse, de préférence des solutions pour pulvérisation nasale, des gouttes nasales ou des solutions pour gargarisme.

3. Utilisation selon la revendication 1 ou 2, **caractérisée en ce que** la préparation comprend des véhicules ou des additifs pharmaceutiques.

4. Utilisation selon l'une quelconque des revendications 1 à 3, **caractérisée en ce que** le polymère est présent en quantité de 0,01 % à 20 %, de préférence de 0,1 % à 10 %, mieux encore de 0,5 % à 5 % de la préparation.

5. Utilisation selon l'une quelconque des revendications 1 à 4, **caractérisée en ce que** la préparation comprend au moins deux composants antiviraux différents, l'un des composants étant de préférence le chitosane.

6. Utilisation selon l'une quelconque des revendications 1 à 5, **caractérisée en ce que** la préparation est stérile.
